# EUROPEAN PATENT APPLICATION

(11) **EP 4 533 997 A1**
(43) Date of publication of application: **09.04.2025**
(21) Application number: 23884106.8
(22) Date of filing: 25.04.2023
(51) Int. Cl.: A61B 1/005

(54) **BENDING PART OF ENDOSCOPE AND ENDOSCOPE**

(30) Priority: 31.10.2022 CN 202211349250
(71) Applicant: Anqing Medical Co., Ltd, Shanghai 201201 (CN)
(72) Inventor: YAN, Hang, Shanghai 201201 (CN); TANG, Wei, Shanghai 201201 (CN)
(74) Representative: Zaboliene, Reda
(86) International application number: PCT/CN2023/090618
(87) International publication number: WO 2024/093164

(57) **Abstract**

The invention provides a bending portion of an endoscope and the endoscope, including: a hollow tube, wherein a plurality of groups of gap portions are disposed on the hollow tube; the plurality of groups of gap portions extend in sequence along an axial direction of the hollow tube; each group of the gap portions includes at least one gap; the gaps are distributed in a circumferential direction of the hollow tube; an included angle of a preset angle is present along the length direction of the gap between a length direction of one end or a length direction of two ends of the gap and a radial direction of the hollow tube; and the radial direction of the hollow tube is perpendicular to the axial direction of the hollow tube. According to the technical solution of the invention, the inclined arrangement of the gaps enhances both the bending softness and the axial support.

## Description

### TECHNICAL FIELD

The invention relates to the technical field of endoscopes, and in particular to a bending portion of an endoscope and the endoscope.

### BACKGROUND ART

Endoscope is a kind of medical and industrial detection device which is widely used. Generally, the snake bone is applied by the portion of endoscope extending into the object to be detected to realize bending and turning.

A plurality of tube sections are cut on a hollow tube by a laser. Each tube section includes a connection unit which cooperates with a connection unit of an adjacent joint to ultimately form a controllable bending portion (commonly known as "snake bone") at the distal end of an endoscope insertion tube.

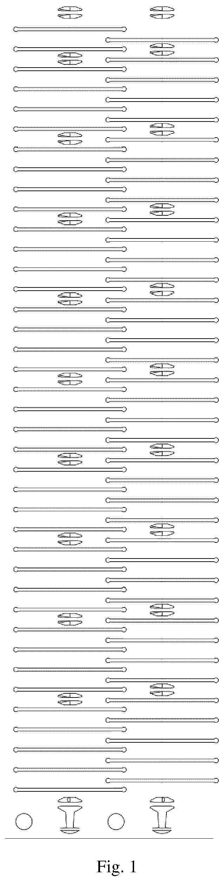

In the prior art, the grooves cut on the hollow tube are all straight grooves perpendicular to the axial direction of the hollow tube. Referring to Fig. 1, this structure has the following disadvantages. On the one hand, in order to satisfy its softness, that is to say, it is easier to bend, and the gap is required to be large enough. However, after the gap is enlarged, the axial support force of the hollow tube will be weakened, and the collapse easily occurs. On the other hand, in order to satisfy the axial support, the gap is required to be smaller. However, the softness of the hollow tube is insufficient after the gap is smaller, and the hollow tube is not easily stretched and bent.

Therefore, the snake bone structure of the prior art cannot satisfy both softness and axial support.

### SUMMARY OF THE INVENTION

The invention provides a bending portion of an endoscope and the endoscope, so as to solve the problems of softness and axial support that cannot be satisfied simultaneously in the prior art.

In order to solve the above technical problem, the invention is achieved by the following technical solutions.

According to a first aspect of the invention, a bending portion of an endoscope is provided, including a hollow tube, wherein a plurality of groups of gap portions are disposed on the hollow tube; the plurality of groups of gap portions extends in sequence along an axial direction of the hollow tube;
each group of the gap portions includes at least one gap; the gaps are distributed in a circumferential direction of the hollow tube;
an included angle of a preset angle is present along the length direction of the gap between a length direction of one end or a length direction of two ends of the gap and a radial direction of the hollow tube; and
the radial direction of the hollow tube is perpendicular to the axial direction of the hollow tube.

Preferably, each group of the gap portions includes two gaps: a first gap and a second gap;
the first gap is distributed in a first circumferential direction of the hollow tube; the second gap is distributed in a second circumferential direction of the hollow tube; and
the first gap and the second gap have a relative position difference of a preset length along the circumferential direction of the hollow tube.

Preferably, the gap is bent around an axial center of the axial direction by a preset arc length; and the positions where the both sides of the gap are contacted are in line contact or surface contact.

Preferably, the gap is curved along the length direction of the gap.

Preferably, the gap is crescent along the length direction of the gap.

Preferably, the hollow tube is further provided with a plurality of groups of wiring groove portions; and the plurality of groups of wiring groove portions extend in sequence in the axial direction of the hollow tube;
each group of the wiring groove portions includes two wiring grooves: a first wiring groove and a second wiring groove; and
the first wiring groove and the second wiring groove are respectively disposed on opposite sides of the hollow tube.

Preferably, the first wiring groove is symmetrical to the second wiring groove.

Preferably, the hollow tube has a wall thickness less than a preset wall thickness value.

Preferably, the preset angle is any value between 10° and 45°.

Preferably, the hollow tube is a hollow steel tube.

According to a second aspect of the invention, an endoscope is provided, including the controllable bending portion of the endoscope described in any one of the above.

According to the bending portion of the endoscope and the endoscope provided in the invention, an included angle of a preset angle is present along the length direction of the gap between a length direction of one end or a length direction of two ends of the gap and a radial direction of the tube, namely, the gap is of an inclined design. When an axial tensile force is applied to the bending portion of the endoscope, one end or both ends of the gap having an included angle generate an acting force in the length direction, and the acting force having an included angle with the radial direction can be decomposed into a radial force and an axial force. The radial force can facilitate the bending, namely, enhancing the bending softness. The axial force can facilitate the axial support, namely, enhancing the axial support.

In an alternative solution of the invention, the gap is bent around an axial center of the axial direction by a preset arc length. The positions where both sides of the gap are contacted are in line contact or surface contact. The contact surface is large, so that the stability of bending can be improved.

In an alternative solution of the invention, the gap is crescent along the length direction of the gap, which not only can better adapt to the inclined design of the gap, but also can bend the gap about the axial axis by a preset arc length. The two sides of the gap are closed by a long curve after contacting, i.e., the contact area of the two sides of the gap after contacting is larger due to bending, and the turning is more stable.

In an alternative aspect of the invention, both softness and axial support are satisfied by the inclined arrangement of the gaps, so that the wall thickness of the hollow tube can be made very thin (less than a preset wall thickness value), the structural size of the endoscope can be effectively improved, and the bending softness can be further enhanced.

### BRIEF DESCRIPTION OF THE DRAWINGS

In order to more clearly describe the technical solutions in the examples of the disclosure or the prior art, the drawings to be used in the description of the examples or the prior art will be briefly introduced below. It will be apparent to those skilled in the art that the drawings in the following description are only some of the disclosure, and that other drawings may be obtained from the drawings without any creative works.
Fig. 1 is an unfolded schematic view of a bending portion of an endoscope in the prior art;
Fig. 2 is a schematic view of a bending portion of an endoscope according to an example of the invention;
Fig. 3 is an expanded view of a bending portion of an endoscope according to an example of the invention; and
Fig. 4 is an expanded view of a bending portion of an endoscope according to a preferred example of the invention;

### Description of Reference Numerals:

1-gap portion,
11-first gap,
12-second gap,
101-destressing incision,
2-wiring groove portion,
21, first wiring groove;
22, second wiring groove;

### DETAILED DESCRIPTION OF THE INVENTION

The technical solutions in the examples of the invention will be described clearly and completely in conjunction with the accompanying drawings in the examples of the invention. Obviously, the described examples are only part of the examples of the invention, rather than all of the examples. Based on the examples in the invention, all other examples obtained by a person skilled in the art without involving any inventive effort are within the scope of protection of the invention.

In the description of the invention, it should be understood that the directional or positional relationships indicated by the terms "upper", "lower", "upper end", "lower end", "lower surface", "upper surface" and the like are based on the directional or positional relationships shown in the drawings. It is merely for the purpose of describing the disclosure and simplifying the invention, and is not intended to indicate or imply that a particular orientation, configuration and operation of the referenced device or element is required and should not be construed as limiting the invention.

In the description of the invention, the terms "first" and "second" are used for descriptive purposes only and are not to be construed as indicating or implying relative importance or implicitly indicating the number of technical features indicated. Thus, the feature defined by "first" or "second" may explicitly or implicitly includes one or more such features.

In the description of the invention, "a plurality of" means multiple, e.g., two, three, four, etc. unless specifically and specifically limited otherwise.

In the invention, unless expressly stated or limited otherwise, the terms "connected" and the like are to be interpreted broadly, e.g., either fixedly or detachably, or integrally modularization. It may be a mechanical connection, an electrical connection, or an intercommunication. It may be a direct connection or indirect connection by an intermediary. It may be a communication between two elements, or may be in an interactive relationship between two elements. The specific meaning of the above terms in this invention will be understood in specific circumstances by those of ordinary skill in the art.

Hereinafter, technical solutions of the invention will be described in detail with reference to specific examples. The following specific examples may be combined with one another, and the same or similar concepts or processes may not be repeated in some examples.

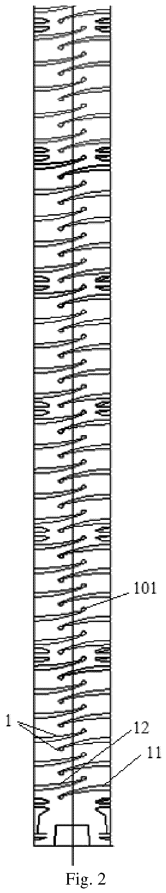

In an example, a bending portion of an endoscope is provided, including a hollow tube, wherein a plurality of groups of gap portions are disposed on the hollow tube; the plurality of groups of gap portions extend in sequence along an axial direction of the hollow tube, as seen in Fig. 2.

Here, each group of the gap portions includes at least one gap; the gaps are distributed in a circumferential direction of the hollow tube. An included angle of a preset angle (namely, an inclined arrangement) is present along the length direction of the gap between a length direction of one end or a length direction of two ends of the gap and a radial direction of the hollow tube. The radial direction of the hollow tube is perpendicular to the axial direction of the hollow tube.

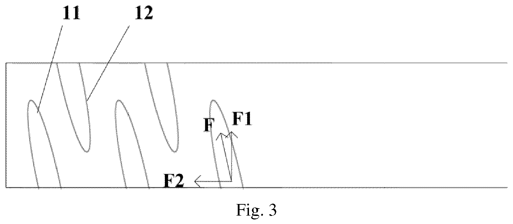

In the above-mentioned examples, an included angle of a preset angle (namely, an inclined arrangement) is present along the length direction of the gap between a length direction of one end or a length direction of two ends of the gap and a radial direction of the tube. When an axial tensile force is applied to the bending portion of the endoscope, one end or both ends of the gap having an included angle generate an acting force F in the length direction, and the acting force having an included angle with the radial direction can be decomposed into a radial force F1 and an axial force F2, as seen in Fig. 3. Here, the radial force F1 can facilitate the bending, i.e., enhance the bending softness. The axial force F2 can facilitate the axial support, i.e., enhance the axial support.

In an example, the axial spacing between adjacent gap portions may be the same or different, and may be set differently according to the bending rigidity requirements of different parts of the bending portion. For example, the axial spacing may be designed to be relatively large for parts with high bending rigidity requirements (e.g., the middle part of the bending portion). The axial spacing may be designed to be relatively small for parts with low bending stiffness (e.g., both ends of the bending portion), .

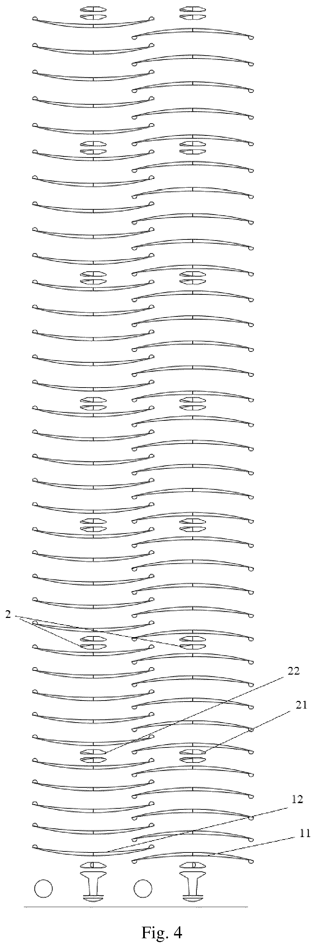

In an example, each set of gap portions includes two gaps: a first gap 11 and a second gap 12, as shown in Figs. 2 and 4. The first gaps 11 are distributed in a first circumferential direction of the hollow tube and the second gaps 12 are distributed in a second circumferential direction of the hollow tube. The first gap 11 and the second gap 12 have a relative position difference of a preset length along the circumferential direction of the hollow tube.

In an example, the gap is bent around an axial center of the axial direction by a preset arc length. The positions where the both sides of the gap are contacted are in line contact or surface contact, so that the stability of rotation may be improved. For specific reasons, the gaps in the prior art are mostly rectangular gaps, i.e., the gap is a rectangular gap, as seen in Fig. 1. When the hollow tube is bent until both sides of the gap are closed, two points must first contact, so that the axial support is insufficient, the bending stability will be weakened while reducing the axial support force, and the hollow tube is easy to swing radially. However, in the invention, when the hollow tube is bent until both sides of the gap are closed, it is the contact of two lines or the contact of two faces, and the contact of two lines or the contact of two faces is smoother than the contact of two points, so as to improve the axial support and thus improve the smoothness of rotation.

In an example, the gap is curved along the length direction of the gap. The gap is bent around an axial center of the axial direction by a preset arc length. The positions where the both sides of the gap are contacted are in line contact or surface contact. The contact area is larger, and the turning is smoother.

In an example, the gap has a crescent shape along the length direction of the gap, as seen in Fig. 3 for an expanded view of the hollow tube. In the crescent-shaped gap, the preset arc length is bent around the axial center of the gap. After the two sides of the gap are in contact with each other, the contact surface is larger, and the rotation is more stable.

In an example, since both ends of the crescent gap are relatively narrow, both ends of the gap are provided with destressing incisions 101 in order to reduce stress, as seen in Fig. 2.

In an example, along the length direction of the gap, the gap may also have other curved gaps, and it may also reach that both sides of the gap are in line contact or in surface contact. For example, a curved gap in the shape of the gap in Fig. 3 may be used.

In an example, the hollow tube is further provided with a plurality of groups of wiring groove portions; and the plurality of groups of wiring groove portions extend in sequence in the axial direction of the hollow tube. Each group of the wiring groove portions includes two wiring grooves: a first wiring groove 21 and a second wiring groove 22, as seen in Fig. 4. The first wiring groove 21 and the second wiring groove 22 are respectively disposed on opposite sides of the hollow tube.

In an example, due to the inclined arrangement of gaps, the first wiring groove 21 and the second wiring groove 22 can be made symmetrical with respect to each other, as seen in Fig. 4. The stress is more uniform and the manufacturing process is simpler. With regard to the design of the vertical gap in the prior art, the wiring grooves cannot be made symmetrical to the left and to the right, only one on the left and one on the right, as seen in Fig. 1. Thus, the machining process is relatively complicated and the stress is uneven.

In an example, both the softness and the axial support are enhanced by the inclined arrangement of gaps. As the axial support is enhanced, the wall thickness of the hollow tube may be made very thin, and the axial support can be satisfied. The wall thickness may be smaller than the preset wall thickness value. The reduced wall thickness of the hollow tube effectively improves both the structural size of the endoscope and the softness of bending.

In an example, the preset angle is any value between 10° and 45°. Preferably, it is more effective for the preset angle between 15° and 25°.

In an example, the softness and the axial support are both enhanced by the inclined arrangement of the gaps. Since the softness is enhanced, the hollow tube may be a hollow steel tube, and it may satisfy the softness even if the tube is a steel tube, and a wiring groove is better formed on the steel tube. In the prior art, in order to satisfy the softness, the steel tube is replaced by the nickel-titanium alloy in some endoscopes. However, the nickel-titanium alloy does not have a wiring groove and is easy to burst.

In an example, an endoscope is also provided, including the bending portion of the endoscope as described in any of the examples above.

In the disclosure of this description, reference to the terms "an embodiment", "an example", and "a specific implementation process", "an instance", etc., means that specific features, structures, materials, or characteristics described in connection with the embodiment or example is included in at least one embodiment or example of the invention. In the present specification, the schematic statement of the above terms does not necessarily refer to the same embodiment or example. Furthermore, the particular features, structures, materials, or characteristics described may be combined in any suitable manner in any one or more embodiments or examples.

Finally, it should be noted that each example above are only intended to illustrate the technical solution of the invention, but not to limit it. Although the invention has been described in detail with reference to the each foregoing example, those skilled in the art will appreciate that the technical solutions of the each above-mentioned example can still be modified, or some of the technical features thereof can be equivalently substituted. Such modifications and substitutions will not cause the essence of the corresponding technical solutions to depart from the scope of the examples of the invention.

## Claims

1. A bending portion of an endoscope, comprising a hollow tube, wherein a plurality of groups of gap portions are disposed on the hollow tube; the plurality of groups of gap portions extend in sequence along an axial direction of the hollow tube;
each group of the gap portions comprises at least one gap; the gaps are distributed in a circumferential direction of the hollow tube;
an included angle of a preset angle is present along the length direction of the gap between a length direction of one end or a length direction of two ends of the gap and a radial direction of the hollow tube; and
the radial direction of the hollow tube is perpendicular to the axial direction of the hollow tube.

2. The bending portion of the endoscope according to claim 1, wherein each group of the gap portions comprises two gaps: a first gap and a second gap;
the first gap is distributed in a first circumferential direction of the hollow tube; the second gap is distributed in a second circumferential direction of the hollow tube; and
the first gap and the second gap have a relative position difference of a preset length along the circumferential direction of the hollow tube.

3. The bending portion of the endoscope according to claim 1, wherein the gap is bent around an axial center of the axial direction by a preset arc length; and the positions where the both sides of the gap are contacted are in line contact or surface contact.

4. The bending portion of the endoscope according to claim 3, wherein the gap is curved along the length direction of the gap.

5. The bending portion of the endoscope according to claim 4, wherein the gap is crescent along the length direction of the gap.

6. The bending portion of the endoscope according to claim 1, wherein the hollow tube is further provided with a plurality of groups of wiring groove portions; and the plurality of groups of wiring groove portions extend in sequence in the axial direction of the hollow tube;
each group of the wiring groove portions comprises two wiring grooves: a first wiring groove and a second wiring groove; and
the first wiring groove and the second wiring groove are respectively disposed on opposite sides of the hollow tube.

7. The bending portion of the endoscope according to claim 6, wherein the first wiring groove is symmetrical to the second wiring groove.

8. The bending portion of the endoscope according to claim 1, wherein the hollow tube has a wall thickness less than a preset wall thickness value.

9. The bending portion of the endoscope according to any one of claims 1 to 8, wherein the preset angle is any value between 10° and 45°.

10. The bending portion of the endoscope according to any one of claims 1 to 8, wherein the hollow tube is a hollow steel tube.

11. An endoscope, comprising the bending portion of the endoscope according to any one of claims 1 to 10.
